(19) Europäisches Patentamt
European Patent Office
Office européen des brevets

(11) **EP 3 264 983 B1**

(12) **FASCICULE DE BREVET EUROPEEN**

(45) Date de publication et mention de la délivrance du brevet:
**23.01.2019 Bulletin 2019/04**

(21) Numéro de dépôt: **16709318.6**

(22) Date de dépôt: **04.03.2016**

(51) Int Cl.:
*A61B 5/113* (2006.01)      *A61B 5/00* (2006.01)

(86) Numéro de dépôt international:
**PCT/EP2016/054706**

(87) Numéro de publication internationale:
**WO 2016/142308 (15.09.2016 Gazette 2016/37)**

(54) **DISPOSITIF DE SURVEILLANCE D'UN PARAMÈTRE PHYSIOLOGIQUE D'UN UTILISATEUR SOUS LA FORME D'UN VÊTEMENT**

VORRICHTUNG IN FORM EINES KLEIDUNGSSTÜCKS ZUR ÜBERWACHUNG EINES PHYSIOLOGISCHEN PARAMETERS EINES BENUTZERS

DEVICE IN THE FORM OF A GARMENT FOR MONITORING A PHYSIOLOGICAL PARAMETER OF A USER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **06.03.2015 FR 1551896**

(43) Date de publication de la demande:
**10.01.2018 Bulletin 2018/02**

(73) Titulaire: **Bioserenity**
**75013 Paris (FR)**

(72) Inventeurs:
• **GOUTHEZ, Marion**
**69230 Saint-Genis-Laval (FR)**
• **ZORMAN, Sylvain**
**75013 Paris (FR)**
• **FROUIN, Pierre-Yves**
**75005 Paris (FR)**

(74) Mandataire: **Regimbeau**
**20, rue de Chazelles**
**75847 Paris Cedex 17 (FR)**

(56) Documents cités:
**EP-A2- 2 505 090          WO-A1-2013/033238**
**JP-A- 2012 040 236       US-A1- 2007 171 024**
**US-A1- 2014 318 699**

## Description

### DOMAINE DE L'INVENTION

L'invention concerne un dispositif de suivi de respiration, sous la forme d'un vêtement pouvant être porté par l'utilisateur.

### ETAT DE LA TECHNIQUE

Afin de suivre en continu l'état physiologique d'un utilisateur, il est connu de fixer des capteurs dans des structures textiles destinées à former un vêtement. De tels capteurs permettent par exemple de mesurer un signal électrocardiographique représentatif de l'activité cardiaque d'un utilisateur qui porte le vêtement.

Cependant, de tels capteurs peuvent parfois s'avérer encombrants, entrainant de ce fait un manque de confort pour l'utilisateur. De plus, l'assemblage de ces capteurs sur le vêtement et leur raccordement électrique peuvent s'avérer complexes.

Le document EP 1 506 738 décrit un vêtement élastique comprenant des capteurs textiles. Les capteurs incluent d'une part des électrodes textiles souples appliquées contre la peau servant à recueillir des signaux électriques engendrés par le corps pour l'élaboration d'un électrocardiogramme ou d'un électromyogramme, et d'autre part, un capteur de déformation mécanique servant à mesurer des mouvements respiratoires de l'utilisateur. Les électrodes sont formées par tissage ou tricotage d'un fil conducteur élastique. Le capteur de déformation mécanique comprend un fil conducteur élastique non-tricoté. Le fil conducteur élastique est obtenu par guipage d'un fil conducteur non extensible autour d'une âme constituée de fil élastique non conducteur, c'est-à-dire que le fil conducteur est enroulé en hélice autour du fil élastique non conducteur. Lorsque le fil conducteur élastique est étiré, le fil s'allonge et les spires voisines de l'enroulement s'écartent les unes des autres, provoquant une variation de résistance électrique mesurable qui est fonction de l'allongement du fil conducteur élastique.

Le document US 2007/0171024 décrit un vêtement dans lequel est intégrée une jauge permettant de suivre la respiration de l'utilisateur qui porte le vêtement. Le vêtement comprend une base textile tissée à partir de fils non-conducteurs. La jauge est formée par un fil conducteur guipé avec un fil non-conducteur, et tissé à travers la base textile, au niveau de la région abdominale ou au niveau de la poitrine de l'utilisateur. Le fil conducteur est constitué de fils métalliques ultrafins torsadés avec des fibres textiles ou de fibres textiles mélangées avec des fibres métalliques. Les mouvements respiratoires de l'utilisateur provoquent un allongement ou une contraction du fil conducteur. La modification de la longueur du fil conducteur entraine une modification des propriétés électriques du fil.

Dans un tel vêtement, le raccordement du fil conducteur à un appareil de mesure nécessite de dénuder au préalable le fil conducteur, c'est-à-dire d'éliminer le fil non-conducteur qui l'entoure, afin de pouvoir réaliser un contact électrique entre le fil conducteur et un câble de raccordement.

En outre, le guipage du fil conducteur avec un fil non conducteur augmente le diamètre total du fil conducteur, ce qui augmente les coûts de fabrication du vêtement et peut créer un inconfort pour l'utilisateur qui porte le vêtement.

Le document FR 2 970 779 ne concerne pas un dispositif de suivi de respiration, mais un dispositif de mesure de pression visant à prévenir l'apparition de plaies de pression chez des personnes dont la mobilité est réduite. Les documents US 2014/0318699, WO 2013/033238, EP 2505090, JP 2012-40236 divulguent des dispositifs de suivi de la respiration intégrés dans un vêtement.

### RESUME DE L'INVENTION

Un but de l'invention est de proposer un dispositif de suivi de la respiration d'un utilisateur, qui présente à la fois un confort amélioré et qui minimise les étapes de confection nécessaires pour la réalisation du dispositif.

Ce but est atteint dans le cadre de la présente invention, définie à la revendication indépendante 1, grâce à un dispositif de suivi de respiration d'un utilisateur comprenant :

- un support textile comprenant une partie tubulaire formée par tricotage d'un fil de fond majoritaire électriquement isolant, la partie tubulaire étant propre à revêtir le buste de l'utilisateur,
- au moins un capteur de respiration formé par tricotage d'un fil de détection, le fil de détection formant une pluralité de mailles, le fil de détection comprenant au moins une âme interne en matériau électriquement isolant et une gaine externe entourant l'âme interne, la gaine externe étant formée en matériau électriquement conducteur de sorte à créer des contacts électriques entre les mailles du fil de détection.

Le capteur de respiration forme une bande conductrice présentant une première extrémité et une deuxième extrémité positionnées à distance l'une de l'autre, les extrémités étant propres à être raccordées à un appareil pour mesurer la résistance électrique de la bande conductrice.

La bande conductrice est positionnée par rapport à la partie tubulaire de sorte que lorsque le buste de l'utilisateur est revêtu du support textile, la bande conductrice est étirée et rétreinte en alternance du fait de la respiration de l'utilisateur, l'étirement et la rétraction de la bande conductrice ayant pour effet de modifier les contacts électriques entre les mailles du fil de détection au sein de la bande conductrice, entrainant une modification de la résistance électrique de la bande conductrice.

Dans un tel dispositif, le fil de détection n'est pas isolé, ce qui autorise l'utilisation d'un fil plus petit, moins coûteux et plus léger. De plus, le raccordement du fil de détection à l'appareil de mesure ne nécessite pas de dénudage préalable du fil de détection.

En outre, les mailles successives du fil de détection forment de multiples contacts de la gaine externe avec elle-même. C'est principalement la modification de ces contacts électriques lors de l'étirement et de la rétraction de la bande conductrice qui génère une modification de la résistance électrique, et non un étirement ou une contraction du fil lui-même.

Le dispositif proposé peut en outre présenter les caractéristiques suivantes :

- le fil de détection comprend au moins une âme en matériau polymère, de préférence en polyamide,
- selon une première possibilité, la gaine du fil de détection est formée par guipage de l'âme interne avec un fil en matériau conducteur, de préférence en argent,
- selon une deuxième possibilité, la gaine du fil de détection est formée par enrobage de l'âme interne avec une couche en matériau conducteur, de préférence en argent,
- le fil peut être constitué de plusieurs filaments non conducteurs, chaque filament étant enrobé avec une couche externe en matériau conducteur, de préférence en argent,
- dans ce cas, les filaments enrobés peuvent être torsadés ensemble,
- la partie tubulaire est formée par tricotage du fil de fond majoritaire et d'un fil élastique,
- la bande conductrice entoure au moins en partie le buste de l'utilisateur, de préférence à hauteur du sternum et/ou des abdominaux, et s'étend sur le ventre et/ou sur le dos de l'utilisateur,
- la bande conductrice peut s'étendre à la fois sur le ventre et sur le dos de l'utilisateur,
- la partie tubulaire et le capteur de respiration sont formés par tricotage circulaire en une seule opération, le fil de fond et le fil de détection étant tricotés en alternance,
- au cours du tricotage, le fil de détection est coupé lorsque le fil de fond est tricoté,
- le dispositif comprend en outre une couche isolante disposée entre la bande conductrice et la peau de l'utilisateur lorsque l'utilisateur est revêtu du support textile,
- le dispositif comprend en outre une gaine fixée sur la partie tubulaire et des câbles de connexion situés à l'intérieur de la gaine pour raccorder les extrémités de la bande conductrice à l'appareil pour mesurer la résistance électrique.

**PRESENTATION DES DESSINS**

D'autres caractéristiques et avantages ressortiront encore de la description qui suit, laquelle est purement illustrative et non limitative et doit être lue en regard des figures annexées, parmi lesquelles :

- la figure 1 représente de manière schématique une vue de devant d'un dispositif de suivi de respiration conforme à un mode de réalisation possible de l'invention,
- la figure 2 représente de manière schématique une vue de derrière du dispositif de suivi de respiration,
- la figure 3 est une vue de détail du dispositif,
- la figure 4 représente de manière schématique le dispositif incluant une gaine et des câbles de connexion,
- les figures 5A et 5B sont des vues schématiques de détail, respectivement de l'envers et de l'endroit du dispositif,
- les figures 6 et 7 représentent de manière schématique un maillage formant un capteur de respiration, lorsque le capteur est à l'état de repos et à l'état étiré respectivement,
- les figures 8 et 9 représentent de manière schématique un fil conducteur formant des mailles, lorsque le fil conducteur est à l'état de repos et à l'état étiré respectivement,
- la figure 10 représente de manière schématique la structure d'un fil non-conducteur utilisé pour former le support textile,
- la figure 11 représente de manière schématique la structure d'un fil conducteur utilisé pour former le capteur selon une première possibilité,
- la figure 12 représente de manière schématique la structure d'un fil conducteur utilisé pour former le capteur selon une deuxième possibilité,
- la figure 13 représente de manière schématique la structure d'un fil conducteur utilisé pour former le capteur selon une troisième possibilité
- la figure 14 est un schéma électrique équivalent du capteur de respiration et d'un appareil pour mesurer la résistance électrique de la bande conductrice,
- la figure 15 représente de manière schématique des variations de résistance électrique enregistrées à partir du capteur de respiration.

**DESCRIPTION DETAILLEE D'UN MODE DE REALISATION**

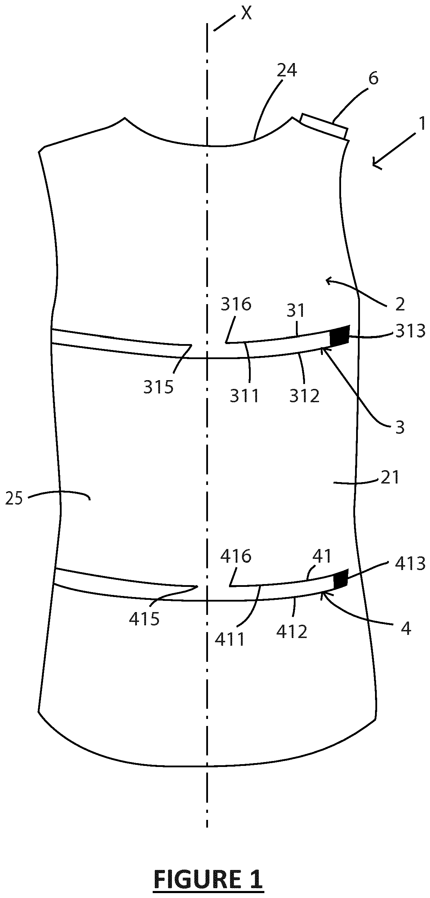

Sur les figures 1 à 4, le dispositif de suivi de respiration 1 représenté comprend un support textile 2 et une pluralité de capteurs 3 et 4 intégrés dans le support textile 2 pour suivre la respiration d'un utilisateur.

Le support textile 2 se présente sous la forme d'un vêtement, tel qu'un tee-shirt par exemple, propre à revêtir le corps de l'utilisateur.

Dans le mode de réalisation illustré sur les figures 1 à 4, les capteurs incluent deux capteurs de respiration 3 et 4.

Les capteurs de respiration 3 et 4 permettent de détecter les mouvements respiratoires de la cage thoracique et/ou de l'abdomen de l'utilisateur qui porte le vêtement.

Le support textile 2 comprend une partie tubulaire 21 propre à entourer le buste de l'utilisateur.

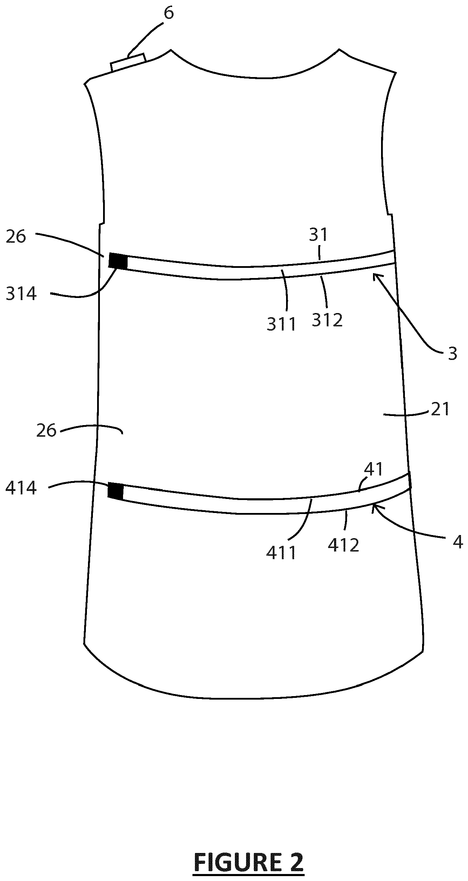

La partie tubulaire 21 présente une encolure 24 pour le passage de la tête, un devant 25 (visible sur la figure 1) propre à recouvrir une partie ventrale de l'utilisateur et un derrière 26 (visible sur la figure 2) propre à recouvrir une partie dorsale de l'utilisateur.

La partie tubulaire 21 est formée par tricotage simultané d'un fil de fond majoritaire, électriquement isolant, et d'un fil élastique.

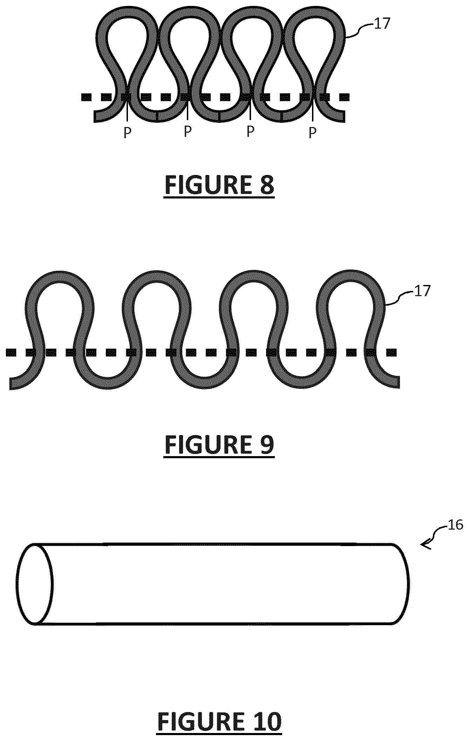

Le fil de fond majoritaire 16 est représenté de manière schématique sur la figure 10. Le fil de fond majoritaire 16 est un fil synthétique, formé en un matériau polymère, tel qu'un fil de polyamide (PA) ou un fil de polyester (PES).

Le fil élastique (non-représenté) est fil formé en élastomère thermoplastique ou un dérivé de polyuréthane, connu sous la marque Lycra® (commercialisé par la société Invista). Le fil élastique peut être guipé ou double-guipé.

Le fil de fond 16 et le fil élastique sont tricotés ensemble de telle façon que le fil de fond recouvre exactement le fil élastique.

Chaque capteur de respiration 3 et 4 s'étend autour du buste de l'utilisateur dans une zone située entre le haut du sternum et le bas du ventre.

Chaque capteur de respiration 3 et 4 est formé par tricotage d'un fil de détection électriquement conducteur.

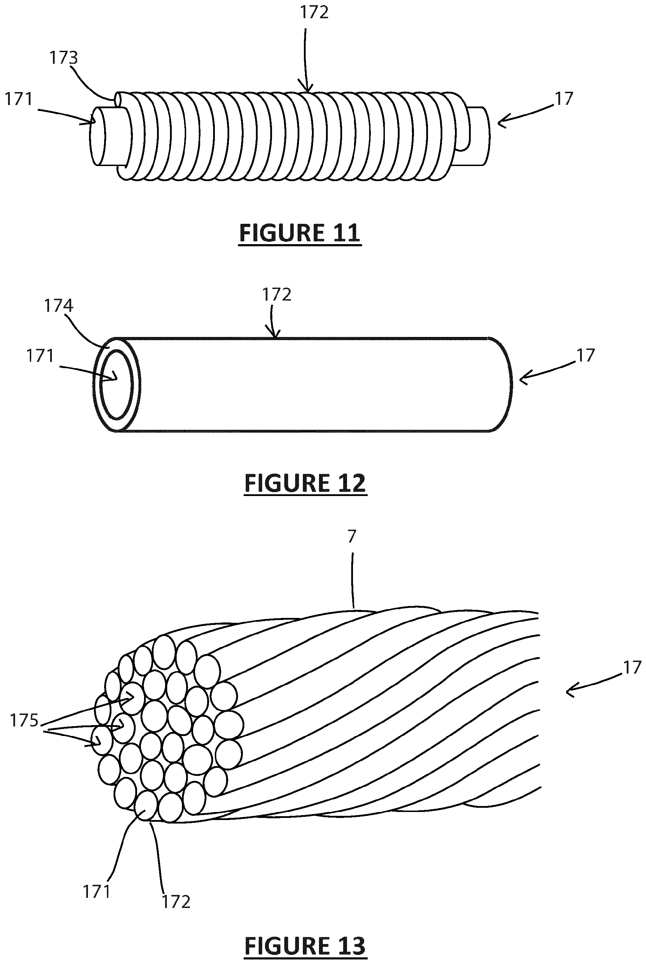

Comme illustré sur les figures 11 à 13, le fil de détection 17 comprend au moins une âme interne 171 en matériau électriquement isolant et une gaine externe 172 entourant l'âme interne, la gaine externe 172 étant formée en matériau électriquement conducteur. Le matériau électriquement isolant de l'âme 171 peut être un polymère, tel que du polyamide par exemple.

Le matériau conducteur de la gaine externe 172 peut être un métal, de préférence un métal biocompatible, tel que l'argent.

Selon une première possibilité (illustrée sur la figure 11), la gaine 172 du fil de détection 17 est formée par guipage de l'âme interne 171 avec un fil 173 en matériau conducteur. Autrement dit, le fil 173 en matériau conducteur est enroulé en hélice autour de l'âme interne 171.

Selon une deuxième possibilité (illustrée sur la figure 12), la gaine 172 du fil de détection est formée par enrobage de l'âme interne 171 avec une couche 174 en matériau conducteur. L'enrobage peut être réalisé par une technique de dépôt sous vide par pulvérisation cathodique par exemple, du matériau conducteur.

Selon une troisième possibilité (illustrée sur la figure 13), le fil de détection 17 est constitué d'un faisceau de filaments conducteurs 175 torsadés ensemble. Chaque filament conducteur comprend une âme 171 en matériau isolant revêtue d'une couche externe 172 en matériau conducteur, de préférence en argent.

Comme cela est visible sur les figures 1 à 4, le premier capteur de respiration 3 forme une première bande conductrice 31 s'étendant autour du buste de l'utilisateur au niveau du sternum, lorsque l'utilisateur porte le vêtement. La première bande conductrice 31 peut comprendre plusieurs branches s'étendant parallèlement les unes aux autres autour du buste de l'utilisateur.

Plus précisément, sur les figures 1 à 4, la première bande conductrice 31 comprend deux branches longitudinales 311, 312 s'étendant parallèlement l'une à l'autre.

Chaque branche longitudinale 311, 312 s'étend suivant la direction de tricotage, c'est-à-dire parallèlement à la direction d'un rang.

De plus, chaque branche longitudinale 311, 312 s'étend à la fois sur le devant et sur le derrière du vêtement.

La première bande 31 comprend également des portions de jonction 313, 314 s'étendant transversalement à la direction de tricotage, et reliant électriquement les branches longitudinales 311, 312 entre elles au niveau de leurs extrémités.

La première branche 311 est interrompue au niveau d'un axe central X du devant du vêtement (axe virtuel passant par le nombril de l'utilisateur).

La première bande 31 forme un circuit électrique présentant deux extrémités 315, 316 situées à distance l'une de l'autre, de part et d'autre de l'axe central X. Les extrémités 315 et 316 de la première bande 31 sont propres à être raccordées électriquement à un appareil pour mesurer la résistance électrique de la première bande conductrice 31.

Chaque branche 311, 312 présente une largeur comprise entre 1 et 50 rangs de mailles.

Le deuxième capteur de respiration 4 forme une deuxième bande conductrice 41 s'étendant autour du buste de l'utilisateur au niveau des abdominaux.

La deuxième bande conductrice 41 est similaire à la première bande conductrice 31. La deuxième bande conductrice 41 comprend deux branches 411, 412 s'étendant parallèlement les unes aux autres autour du buste de l'utilisateur et deux portions de jonction 413, 414. La première branche 411 est interrompue au niveau d'un axe central X. La deuxième bande conductrice 41 présente ainsi également deux extrémités 415 et 416 situées à distance l'une de l'autre, de part et d'autre de l'axe X, et propres à être raccordées électriquement à un appareil pour mesurer la résistance électrique de la deuxième bande conductrice 41.

La partie tubulaire 21 et les capteurs de respiration 3 et 4 sont formés par tricotage circulaire en une seule opération. L'insertion des fils conducteurs est dite « par le biais de la broderie ».

Ainsi, le fil de fond 16 (avec le fil élastique) formant la partie principale tubulaire 21 et le fil de détection 17 formant les capteurs 3 et 4 sont tricotés en alternance pendant l'opération de tricotage.

Autrement dit, pendant l'opération de tricotage, le fil de fond 16 cesse d'être tricoté lorsque le fil de détection 17 est tricoté. De même, le fil de détection 17 cesse d'être tricoté lorsque le fil de fond 16 est tricoté.

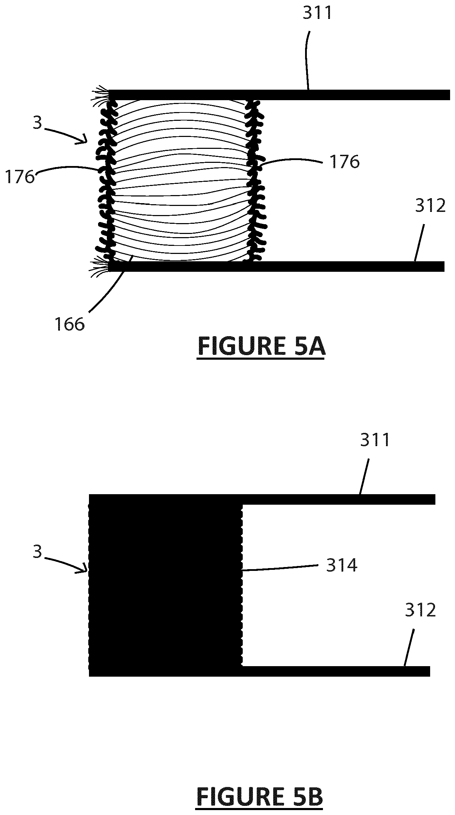

De plus, comme illustré sur les figures 5A et 5B, le fil de détection 17 est coupé lorsque le fil de fond 16 est tricoté. Sur la figure 5A, les extrémités coupées 176 du fil de détection 17 apparaissent sur l'envers du vêtement le long des bords des portions de jonction 313, 314 (de même le long des bords des portions de jonction 413, 414) des capteurs de respiration.

En revanche, le fil de fond 16 n'est pas coupé de sorte que des portions non-tricotées 166 du fil de fond apparaissent sur l'envers du vêtement derrière les portions de jonction 313, 314 (de même le long des bords des portions de jonction 413, 414) des capteurs de respiration.

Par ailleurs, le dispositif 1 de suivi de respiration comprend une ou plusieurs poches fixées sur le support textile par sertissage d'une pièce métallique type boutons de pression, oeillets ou rivets, par couture, soudure ou thermocollage. Ces poches additionnelles permettent l'insertion de composants électroniques dans le vêtement, tels qu'une batterie ou un appareil de mesure par exemple. Afin de limiter le déplacement des composants électroniques par rapport au support textile, les dimensions de chaque poche sont inférieures aux dimensions du composant qu'elle reçoit. L'insertion du composant dans la poche est possible du fait des propriétés élastiques du support textile.

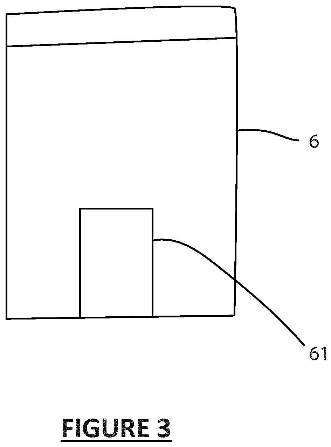

En particulier, le dispositif 1 comprend une poche 6 (visible sur les figures 1 à 4) et un appareil de mesure de résistance 61 (visible sur la figure 3) logé dans la poche 6. La poche 6 est positionnée sur le support textile 2 de manière à être situé sur une épaule de l'utilisateur lorsque l'utilisateur porte le vêtement. Cette position permet de minimiser la gêne créée par la présence de l'appareil lorsque l'utilisateur est allongé. L'appareil de mesure de résistance 61 est propre à mesurer et enregistrer les variations de résistance des bandes conductrices 31 et 41, afin de suivre la respiration de l'utilisateur.

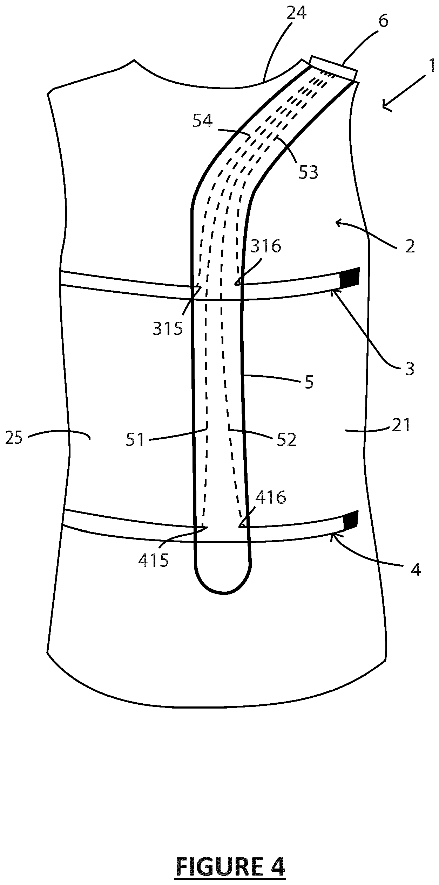

Le dispositif 1 de suivi de respiration comprend en outre une gaine centrale 5 (visible sur la figure 4) fixée sur la partie tubulaire 21 le long de l'axe central X et des câbles de connexion électriques 51 à 54 situés à l'intérieur de la gaine 5 pour raccorder chacun des capteurs à l'appareil de mesure 61. La gaine centrale s'étend le long de l'axe central X du vêtement. La gaine centrale 5 est fixée de préférence sur l'envers du dispositif.

Le raccordement des capteurs de respiration 3 et 4 est réalisé de la manière suivante. Une extrémité dénudée d'un câble de connexion 51, 52, 53, 54 est prise en sandwich entre une extrémité 315, 316, 415, 416 à raccorder et une pièce de textile conductrice rapportée. La pièce de textile est fixée par collage sur l'extrémité 315, 316, 415, 416 à l'aide d'une colle. La colle utilisée est par exemple une colle à base de polyprocaprolactone (PCL).

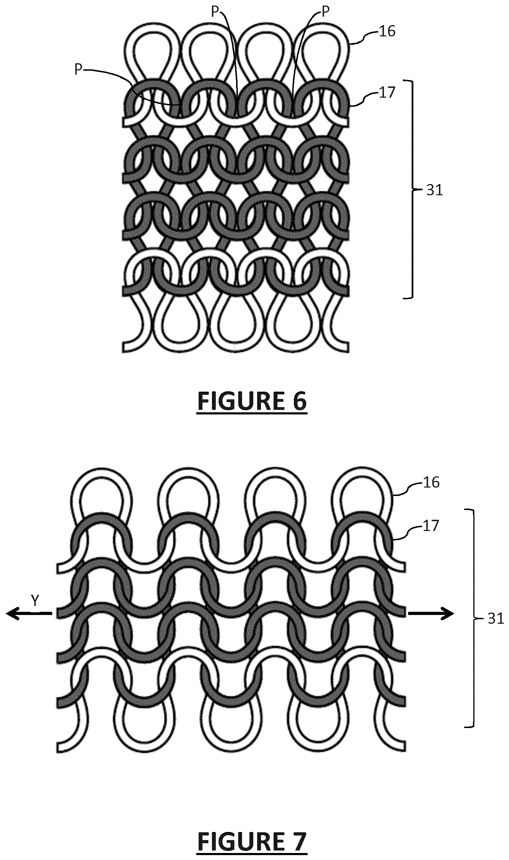

Les figures 6 et 7 illustrent de manière schématique la structure de la bande conductrice 31 formant le capteur de respiration 3, respectivement à l'état de repos et à l'état étiré.

Le fil de fond 16 est tricoté de manière à former une pluralité de rangs.

De même, le fil de détection 17 est tricoté de manière à former une pluralité de rangs.

La technique de tricotage utilisée pour le fil de détection 17 est un tricotage à maille cueillie (c'est-à-dire que les mailles formées par un même fil continu sont disposées dans un même rang), de préférence base Jersey. La même technique de tricotage peut être utilisée pour le fil de fond 16.

Chaque rang est composé d'une pluralité de mailles successives. Les mailles d'un même rang forment des boucles courbées alternativement dans un sens puis dans l'autre, de sorte que les mailles du rang sont entrelacées alternativement avec les mailles du rang immédiatement inférieur et avec les mailles du rang immédiatement supérieur.

Comme illustré sur la figure 6, lorsque la bande conductrice 31 est à l'état de repos, les mailles d'un même rang sont en contact les unes avec les autres en une pluralité de points de contact P.

Comme illustré sur la figure 7, lorsque la bande conductrice 31 est étirée dans une direction Y parallèle à la direction de tricotage (c'est-à-dire la direction d'un rang), les mailles du fil de détection 17 s'écartent les unes des autres, ce qui réduit le nombre de points de contact P entre les mailles.

L'écartement des mailles entraine ainsi une reconfiguration des points de contact P au sein de la bande conductrice 31, ce qui a pour effet de modifier la résistance électrique de la bande conductrice 31.

Plus précisément, comme cela est illustré sur la figure 8, lorsque la bande conductrice 31 est à l'état de repos, un courant électrique peut circuler le long du fil de détection 17 via les points de contact P le long de la ligne en traits pointillés.

En revanche, comme cela est illustré sur la figure 9, lorsque la bande conductrice 31 est étirée, ces points de contact disparaissent, ce qui augmente la résistance électrique effective du fil de détection 17.

Ainsi, en mesurant les variations de résistance électrique de la bande conductrice 31, il est possible de détecter les mouvements respiratoires de l'utilisateur.

Le même principe s'applique à la bande conductrice 41.

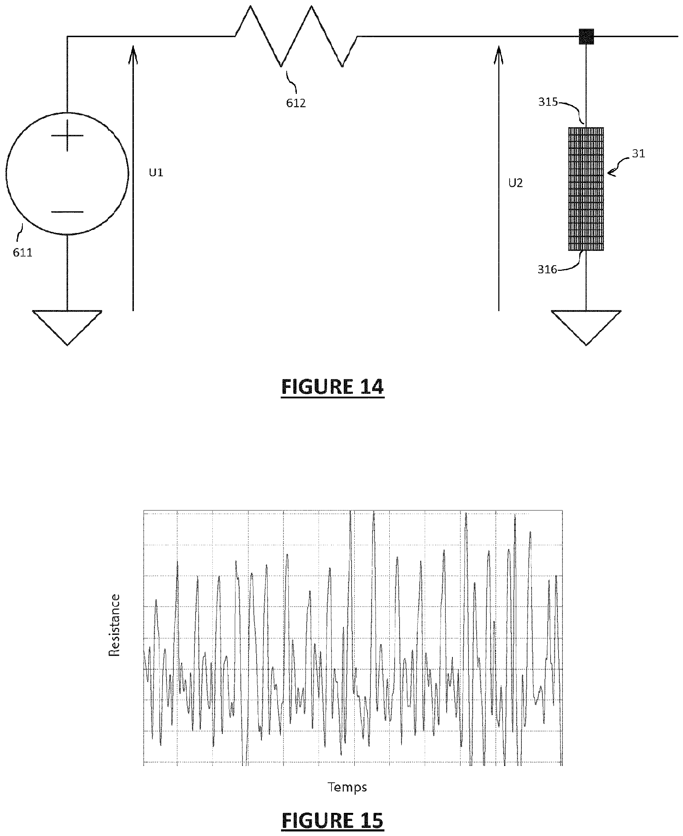

La figure 14 est un schéma de principe d'un circuit électrique d'un appareil 61 pour mesurer la résistance électrique de la bande conductrice 31.

L'appareil 61 comprend un générateur de tension 611, et une première résistance 612. Le générateur de tension 611 génère une tension d'entrée U1 faible, de l'ordre de 10 millivolts.

La tension de sortie U2 générée aux bornes de la bande conductrice est égale à :

$$U2 = U1* R / (R + R612)$$

où R est la résistance de la bande conductrice 31, R612 est la valeur de la résistance 612 et U1 est la tension d'entrée générée par le générateur 611.

En mesurant la tension entre les extrémités 315 et 316 de la bande conductrice 31, il est possible de déduire la résistance R de la bande conductrice 31.

La figure 15 est un diagramme représentant les variations de la résistance de la bande conductrice 31 mesurée au cours du temps lorsque le dispositif 1 est porté par un utilisateur en train de respirer.

La résistance de la bande conductrice 31 est directement fonction de son allongement. Les variations de résistance mesurées peuvent être traitées afin de surveiller des paramètres de respiration, tels que le rythme respiratoire de l'utilisateur ou l'amplitude des cycles respiratoires.

Le dispositif 1 peut comprendre en outre une ou plusieurs couche(s) isolante(s) (non-représentée(s)), disposée(s) entre la ou les bande(s) conductrice(s) 31 et 41, et la peau de l'utilisateur lorsque l'utilisateur est revêtu du support textile 2. La ou les couche(s) isolante(s) peuvent être formées d'un tissu ou d'une membrane extensible. La ou les couche(s) isolante(s) permettent d'isoler électriquement la peau de l'utilisateur des bandes conductrices 31 et 41 dans lesquelles le courant électrique circule.

**Revendications**

1. Dispositif de suivi (1) de la respiration d'un utilisateur comprenant :

   - un support textile (2) comprenant une partie tubulaire (21) formée par tricotage d'un fil de fond (16) majoritaire électriquement isolant, la partie tubulaire (21) étant propre à revêtir le buste de l'utilisateur,
   - au moins un capteur de respiration (3, 4) comprenant un fil de détection (17), le fil de détection (17) comprenant au moins une âme interne (171) en matériau électriquement isolant et une gaine externe (172) entourant l'âme interne (171), la gaine externe (172) étant formée en matériau électriquement conducteur,

   dans lequel le capteur de respiration (3, 4) forme une bande conductrice (31, 41) présentant une première extrémité (311, 411) et une deuxième extrémité (312, 412) positionnées à distance l'une de l'autre, les extrémités étant propres à être raccordées à un appareil pour mesurer la résistance électrique de la bande conductrice (31, 41),
et la bande conductrice (31, 41) est positionnée par rapport à la partie tubulaire (21) de sorte que lorsque le buste de l'utilisateur est revêtu du support textile (2), la bande conductrice (31, 41) est étirée et rétreinte en alternance du fait de la respiration de l'utilisateur, ,
**caractérisé en ce que**
le capteur de respiration est formé par tricotage du fil de détection (17), le fil de détection (17) formant une pluralité de mailles, la gaine externe (172) du fil de détection (17) créant des contacts électriques entre les mailles, de sorte que l'étirement et la rétraction de la bande conductrice (31, 41) a pour effet de modifier les contacts électriques entre les mailles du fil de détection (17) au sein de la bande conductrice (31, 41), entrainant une modification de la résistance électrique de la bande conductrice (31, 41).

2. Dispositif selon la revendication 1, dans lequel le fil de détection (17) comprend au moins une âme (171) en matériau polymère, de préférence en polyamide.

3. Dispositif selon l'une des revendications 1 et 2, dans lequel la gaine (172) du fil de détection (17) est formée par guipage de l'âme interne (171) avec un fil en matériau conducteur, de préférence en argent.

4. Dispositif selon l'une des revendications 1 et 2, dans lequel la gaine (171) du fil de détection (17) est formée par enrobage de l'âme interne (171) avec une couche en matériau conducteur, de préférence en argent.

5. Dispositif selon l'une des revendications 1 à 4, dans lequel le fil de détection (17) est constitué de plusieurs filaments conducteurs (175), chaque filament conducteur (175) comprenant une âme (171) enrobé avec une couche externe (172) en matériau conducteur, de préférence en argent.

6. Dispositif selon la revendication 5, dans lequel les filaments enrobés (175) sont torsadés ensemble.

7. Dispositif selon l'une des revendications 1 à 6, dans lequel la partie tubulaire (21) est formée par tricotage du fil de fond (16) majoritaire et d'un fil élastique.

8. Dispositif selon l'une des revendications 1 à 7, dans lequel la bande conductrice (31, 41) entoure le buste ou une partie du buste de l'utilisateur, de préférence à hauteur du sternum et/ou des abdominaux, et s'étend sur la partie ventrale et/ou sur la partie dorsale de l'utilisateur.

9. Dispositif selon l'une des revendications 1 à 8, dans lequel la partie tubulaire (21) et le capteur de respiration (3, 4) sont formés par tricotage circulaire en une seule opération, le fil de fond (16) et le fil de détection (17) étant tricotés en alternance.

10. Dispositif selon la revendication 9, dans lequel, au cours du tricotage, le fil de détection (17) est coupé lorsque le fil de fond est tricoté (16).

11. Dispositif selon l'une des revendications 1 à 10, comprenant en outre une couche isolante disposée entre la bande conductrice (31, 41) et la peau de l'utilisateur lorsque l'utilisateur est revêtu du support textile (2).

12. Dispositif selon l'une des revendications 1 à 10, comprenant en outre une gaine fixée sur la partie tubulaire (21) et des câbles de connexion situés à l'intérieur de la gaine pour raccorder les extrémités (315, 316, 415, 416) de la bande conductrice (31, 41) à l'appareil pour mesurer la résistance électrique.

**Patentansprüche**

1. Vorrichtung (1) zur Überwachung der Atmung eines Benutzers, umfassend:

- einen Textilträger (2), der einen schlauchförmigen Teil (21) umfasst, der durch Stricken eines mehrheitlichen elektrisch isolierenden Grundgarns (16) gebildet ist, wobei der schlauchförmige Teil (21) geeignet ist, die Brust des Benutzers zu bedecken,
- zumindest einen Respirationssensor (3, 4), der ein Erkennungsgarn (17) umfasst, wobei das Erkennungsgarn (17) zumindest einen inneren Kern (171) aus elektrisch isolierendem Material und einen den inneren Kern (171) umgebenden Außenmantel (172) umfasst, wobei der Außenmantel (172) aus elektrisch leitfähigem Material gebildet ist,

wobei der Respirationssensor (3, 4) einen Leiterstreifen (31, 41) mit einem ersten Ende (311, 411) und einem zweiten Ende (312, 412) bildet, die in einem Abstand voneinander angeordnet sind, wobei die Enden geeignet sind, mit einem Gerät zum Messen des elektrischen Widerstands des Leiterstreifens (31, 41) verbunden zu werden, und der Leiterstreifen (31, 41) gegenüber dem schlauchförmigen Teil (21) so positioniert ist, dass, wenn die Brust des Benutzers mit dem Textilträger (2) bedeckt ist, der Leiterstreifen (31, 41) durch die Atmung des Benutzers abwechselnd gedehnt und geschrumpft wird, **dadurch gekennzeichnet, dass**

der Respirationssensor durch Stricken des Erkennungsgarns (17) gebildet wird, wobei das Erkennungsgarn (17) eine Mehrzahl von Maschen bildet, wobei der Außenmantel (172) des Erkennungsgarns (17) elektrische Kontakte zwischen den Maschen erzeugt, so dass das Dehnen und Schrumpfen des Leiterstreifens (31, 41) die Wirkung hat, die elektrischen Kontakte zwischen den Maschen des Erkennungsgarns (17) innerhalb des Leiterstreifens (31, 41) zu verändern, wodurch eine Änderung des elektrischen Widerstands des Leiterstreifens (31, 41) bewirkt wird.

2. Vorrichtung nach Anspruch 1, wobei das Erkennungsgarn (17) zumindest einen Kern (171) aus Polymermaterial, vorzugsweise Polyamid, umfasst.

3. Vorrichtung nach einem der Ansprüche 1 und 2, wobei der Mantel (172) des Erkennungsgarns (17) durch Umspinnung des inneren Kerns (171) mit einem Garn aus leitfähigem Material, vorzugsweise Silber, gebildet wird.

4. Vorrichtung nach einem der Ansprüche 1 und 2, wobei der Mantel (172) des Erkennungsgarns (17) durch Beschichtung des inneren Kerns (171) mit einer Schicht aus leitfähigem Material, vorzugsweise Silber, gebildet ist.

5. Vorrichtung nach einem der Ansprüche 1 bis 4, wobei das Erkennungsgarn (17) aus mehreren leitenden Fasern (175) besteht, wobei jede leitende Faser (175) einen Kern (171) umfasst, der mit einer Außenschicht (172) aus leitfähigem Material, vorzugsweise Silber, beschichtet ist.

6. Vorrichtung nach Anspruch 5, wobei die beschichteten Fasern (175) miteinander verdrillt sind.

7. Vorrichtung nach einem der Ansprüche 1 bis 6, wobei der schlauchförmige Teil (21) durch Stricken eines mehrheitlichen elektrisch isolierenden Grundgarns (16) und eines elastischen Garns gebildet wird.

8. Vorrichtung nach einem der Ansprüche 1 bis 7, wobei der Leiterstreifen (31, 41) die Brust oder einen Teil der Brust des Benutzers, vorzugsweise auf Höhe des Brustbeins und/oder der Bauchmuskulatur umgibt, und sich über den ventralen Teil und/oder den Rückenteil des Benutzers erstreckt.

9. Vorrichtung nach einem der Ansprüche 1 bis 8, wobei der schlauchförmige Teil (21) und der Respirationssensor (3, 4) durch Rundstricken in einem einzigen Arbeitsgang gebildet werden, wobei das Grundgarn (16) und das Erkennungsgarn (17) abwechselnd gestrickt werden.

**10.** Vorrichtung nach Anspruch 9, bei der während des Strickens das Erkennungsgarn (17) geschnitten wird, wenn das Grundgarn gestrickt wird (16).

**11.** Vorrichtung nach einem der Ansprüche 1 bis 10, ferner umfassend eine Isolierschicht, die zwischen dem Leiterstreifen (31, 41) und der Haut des Benutzers angeordnet ist, wenn der Benutzer mit dem Textilträger (2) bedeckt ist.

**12.** Vorrichtung nach einem der Ansprüche 1 bis 10, ferner umfassend einen auf dem schlauchförmigen Teil (21) befestigten Mantel und Verbindungskabel, die im Innern des Mantels angeordnet sind, um die Enden (315, 316, 415, 416) des Leiterstreifens (31, 41) mit dem Gerät zum Messen des elektrischen Widerstands zu verbinden.

**Claims**

**1.** A device for monitoring (1) the breathing of a user comprising:

- a textile support (2) comprising a tubular portion (21) formed by knitting an electrically insulating majority ground yarn (16), the tubular portion (21) being able to cover the chest of the user,
- at least one breathing sensor (3, 4) comprising a detection yarn (17), the detection yarn (17) comprising at least one internal core (171) made of an electrically insulating material and an external sheath (172) surrounding the internal core (171), the external sheath (172) being made of an electrically conducting material,

wherein the breathing sensor (3, 4) forms a conductive band (31, 41) having a first end (311, 411) and a second end (312, 412) positioned at a distance from each other, the ends being able to be connected to an apparatus for measuring the electric resistance of the conductive band (31, 41), and the conductive band (31, 41) is positioned with respect to the tubular portion (21) so that when the chest of the user is covered with the textile support (2), the conductive band (31, 41) is alternatively stretched and shrunk because of the breathing of the user,
**characterized in that**
the breathing sensor is formed by knitting the detection yarn (17), the detection yarn (17) forming a plurality of stitches, the external sheath (172) of the detection yarn (17) generating electric contacts between the stitches, so that the stretching and the shrinking of the conductive band (31, 41) having the effect of modifying the electric contacts between the stitches of the detection yarn (17) within the conductive band (31, 41), causing a modification in the electric resistance of the conductive band (31, 41).

**2.** The device according to claim 1, wherein the detection yarn (17) comprises at least one core (171) made of a polymeric material, preferably of polyamide.

**3.** The device according to one of claims 1 and 2, wherein the sheath (172) of the detection yarn (17) is formed by wrapping the internal core (171) with a yarn made of a conductive material, preferably of silver.

**4.** The device according to one of claims 1 and 2, wherein the sheath (172) of the detection yarn (17) is formed by coating the internal core (171) with a layer made of a conductive material, preferably of silver.

**5.** The device according to one of claims 1 to 4, wherein the detection yarn (17) consists of several conductive filaments (175), each conductive filament (175) comprising a core (171) coated with an external layer (172) made of a conductive material, preferably of silver.

**6.** The device according to claim 5, wherein the coated filaments (175) are twisted together.

**7.** The device according to one of claims 1 to 6, wherein the tubular portion (21) is formed by knitting the majority ground yarn (16) and an elastic yarn.

**8.** The device according to one of claims 1 to 7, wherein the conductive band (31, 41) surrounds the chest or a portion of the chest of the user, preferably at the height of the sternum and/or the abdominal muscles, and extends over the belly portion and/or over the back portion of the user.

**9.** The device according to one of claims 1 to 8, wherein the tubular portion (21) and the breathing sensor (3, 4) are formed by circular knitting in a single operation, the ground yarn (16) and the detection yarn (17) being alternately knitted.

**10.** The device according to claim 9, wherein, during the knitting, the detection yarn (17) is cut when the ground yarn is knitted (16).

**11.** The device according to one of claims 1 to 10, further comprising an insulating layer positioned between the conductive band (31, 41) and the skin of the user when the user is covered with the textile support (2) .

**12.** The device according to one of claims 1 to 10, further comprising a sheath attached on the tubular portion (21) and connecting cables located inside the sheath for connecting the ends (315, 316, 415, 416) of the conductive band (31, 41) to the apparatus for measuring the electric resistance.

X

24    6

1

2

316    31

315    313

311    312    3

21

25

416    41    413

415    412    4

411

**FIGURE 1**

**FIGURE 2**

**FIGURE 3**

**FIGURE 4**

311

3

176

176

312

166

**FIGURE 5A**

311

3

314

312

**FIGURE 5B**

**FIGURE 6**

**FIGURE 7**

**FIGURE 8**

**FIGURE 9**

**FIGURE 10**

**FIGURE 11**

**FIGURE 12**

**FIGURE 13**

**FIGURE 14**

Temps

**FIGURE 15**

**EP 3 264 983 B1**

## RÉFÉRENCES CITÉES DANS LA DESCRIPTION

**Documents brevets cités dans la description**

- EP 1506738 A
- US 20070171024 A
- FR 2970779
- US 20140318699 A
- WO 2013033238 A
- EP 2505090 A
- JP 2012040236 A